# EUROPEAN PATENT APPLICATION

(11) **EP 1 132 398 A2**
(43) Date of publication of application: **12.09.2001**
(21) Application number: 01301728.0
(22) Date of filing: 26.02.2001
(51) Int. Cl.: C07K 14/255, C12N 15/31, C12N 15/10, A61K 39/112

(54) **DNA sequence encoding the specific and antigenic outer membrane protein of salmonella typhi**

(30) Priority: 28.02.2000 MY 0000765
(71) Applicant: UNIVERSITI SAINS MALAYSIA, 11800 Pulau Pinang (MY)
(72) Inventor: Ismail, Asma Binti, 11800 Pulau, Pinang (MY); Hai, Ong Kok, 59100 Bangsar Kuala Lumpur (MY); Ravichandran, Manickam, 11800 Pulau, Pinang (MY); Zainoodin, S.A. Kader, 11800 Pulau, Pinang (MY)
(74) Representative: Pidgeon, Robert John

(57) **Abstract**

The genetic material encoding for a specific outer membrane protein (OMP) of *Salmonella typhi has* been isolated and characterised. This genetic material (ST50) allows for the production of specific proteins/peptides/DNA/RNA for its use in diagnostics, detection of the bacteria *S.typhi* or in the production of vaccines for typhoid fever.

## Description

### FIELD OF INVENTION

This invention relates to the field of genetic engineering, diagnostics and vaccinology in relation to the gene encoding for the specific OMP of S. *typhi (ST50).* The protein is estimated to have a molecular weight of 50kDa. This protein is available as a result of genetic engineering of the gene that encodes it.

### DESCRIPTION OF THE BACKGROUND

Typhoid fever remains a public health problem in most developing countries. The available conventional methods for the diagnosis of the disease remain unsatisfactory since they are too slow to allow quick decision by the clinician. Culture method may show specificity but it lacked sensitivity and speed. It produced results within 2-7 days and cases of culture negative typhoid were well recognised. The antibody detection test (Widal test) although widely used, lacked speed, sensitivity and specificity. For meaningful interpretation of the test, demonstration of 4-fold rise in antibody titers between acute and convalescent sera 10-14 days later was essential. An ideal diagnostic test for typhoid should be rapid, easy to perform sensitive as well as specific. Neither of the above methods mentioned above satisfied the criteria. Thus there is a need to develop a rapid and specific test. Combined with sensitive diagnosis, the test would provide for prompt, effective and definite management of typhoid fever. In line with the rapid diagnosis for typhoid fever, we have previously made a significant breakthrough in typhoid diagnosis with the discovery of the 50kDa OMP that is specific for the aetiologic agent, *Salmonella typhi* (Ismail A, et *al.* 1991 Biochem Biophys Res Commun 27:301-5). The discovery of the specific 50kDa antigen in the outer membrane of *Salmonella typhi* has been patented in Malaysia (Malaysian patent No: MY-106708-A). Using the isolated OMP, we have successfully developed a rapid dot EIA test (*TYPHIDOT* ™) to detect for the presence of specific IgM and IgG antibodies to the bacteria. The test has a sensitivity of > 95%, a high negative predictive value and could produce results within 1-3 hours. The detection of IgM alone or with IgG would suggest acute typhoid while the detection of IgG only posed several interpretations such as convalescence or possible re-infection. Due to the lack of effective immunity to typhoid fever, patients in highly endemic areas often have re-infections. In the event of current re-infection, there will be a secondary immune response with a significant "boosting" effect of IgG over IgM such that the latter may not be detected. A possible strategy to resolve this problem is to "unmask" the presence of IgM by removing total IgG. The development of a 3 hour IgM detection test (*TYPHIDOT-M ™*) is useful in areas of high endemicity since it could differentiate new from convalescent cases. Data from studies to demonstrate the sensitivity, specificity and advantages of the various tests compared to conventional methods showed that the tests provided reliable alternatives to the Widal test.

In order to produce the protein on a large scale, we have purified and determined the amino acid sequence of the 50kDa OMP. Based on this finding we have isolated, cloned and sequenced the DNA encoding for the 50kDa OMP. Further we have identified the immunogenic epitope of the protein by epitope mapping. This epitope has been used successfully for the upscaling of the 50kDa OMP in the diagnosis of typhoid fever.

### SUMMARY OF THE INVENTION

The present invention provides genetic material encoding for the 50kDa OMP of *Salmonella typhi*. The genetic material can be used to produce sufficient quantities of proteins to be used in diagnostic methods for typhoid fever. Additionally, the genetic material can be used as a probe for the detection of *S.typhi*. The entire protein or epitopes derived from the protein can be used for the diagnosis and vaccine development for typhoid fever. Further use includes development of DNA/RNA vaccines for typhoid fever.

### DESCRIPTION OF DRAWINGS

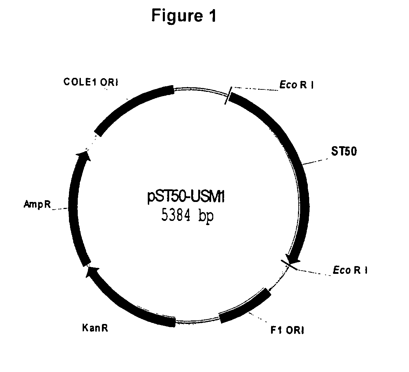

Figure 1 is a plasmid map of ST50 clone in TOPO 2.1 cloning vector. The antibiotic markers ampicillin (AmpR), kanamycin (KanR), origin of replication sites COLE1 and F10RI and the ST50 gene is labeled in the map. The size of the clone is 5384bp.

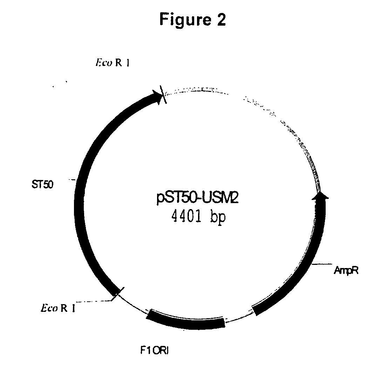

Figure 2 is a plasmid map of ST50 clone in pRSETB expression vector. The antibiotic markers ampicillin (AmpR), origin of replication sites F10RI and the ST50 gene is labeled in the map. The size of the clone is 4401bp.

### DESCRIPTION OF SPECIFIC EMBODIMENTS

The present inventors have identified and obtained for the first time the genetic material encoding for the specific OMP of *S.typhi*, which previously has been available only in limited quantities. Since the 50kDa OMP is central to the detection of specific IgM and IgG antibodies and other antibody classes, availability of the protein in significant quantities in pure form will allow the design of specific diagnostics for typhoid fever. Further, identification and isolation of this specified genetic material allows for production of new vaccines for typhoid. It also provides a source for specific nucleic acid probes for use in hybridization techniques that allow direct detection of *S.typhi*. The first residue of the predicted start codon is designated nucleotide 7 and the entire sequence is presented in Table 1.

### Table 1

### Salmonella typhi ST50 gene DNA and amino acid sequence:

The nucleotide sequences are represented in triplets with their position numbers. The open reading frame (ORF) starts at 7bp nucleotide position with a start codon "ATG" and stops with a stop codon "TGA" at 1474bp nucleotide position. The amino acid coded by the DNA sequence is represented below the DNA sequence with their position numbers. The signal sequence of the ST50 protein is a 16 amino acid peptide that starts at 3^{rd} aminoacid position and ends at 18^{th} amino acid position.

1 ATG CAA ATG AAG AAA TTG CTC CCC ATC CTT ATC GGC CTG AGC CTG 45
1 Met Gln Met Lys Lys Leu Leu Pro Ile Leu Ile Gly Leu Ser Leu 15

46 TCG GGG TTC AGC ACA CTA AGC CAG GCA GAG AAC CTG ATG CAA GTT 90
16 Ser Gly Phe Ser Thr Leu Ser Gln Ala Glu Asn Leu Met Gln Val 30

91 TAT CAG CAA GCA CGC CTG AGC AAC CCG GAA TTG CGT AAA TCC GCT 135
31 Tyr Gln Gln Ala Arg Leu Ser Asn Pro Glu Leu Arg Lys Ser Ala 45

136 GCC GAT CGC GAT GCT GCA TTC GAA AAA ATT AAC GAA GCG CGT AGT 180
46 Ala Asp Arg Asp Ala Ala Phe Glu Lys Ile Asn Glu Ala Arg Ser 60

181 CCT TTA CTG CCG CAA CTG GGT TTA GGT GCC GAC TAC ACC TAC AGC 225
61 Pro Leu Leu Pro Gln Leu Gly Leu Gly Ala Asp Tyr Thr Tyr Ser 75

226 AAC GGT TAT CGC GAT GCG AAC GGT ATC AAC TCC AAT GAA ACC AGC 270
76 Asn Gly Tyr Arg Asp Ala Asn Gly Ile Asn Ser Asn Glu Thr Ser 90

271 GCT TCT CTG CAA TTA ACG CAG ACG CTA TTT GAT ATG TCG AAA TGG 315
91 Ala Ser Leu Gln Leu Thr Gln Thr Leu Phe Asp Met Ser Lys Trp 105

316 CGT GGG CTC ACC CTG CAA GAA AAA GCA GCA GGC ATT CAG GAT GTC 360
106 Arg Gly Leu Thr Leu Gln Glu Lys Ala Ala Gly Ile Gln Asp Val 120

361 ACC TAT CAG ACC GAT CAG CAG ACG CTG ATC CTC AAT ACC GCG AAC 405
121 Thr Tyr Gln Thr Asp Gln Gln Thr Leu Ile Leu Asn Thr Ala Asn 135

406 GCG TAT TTT AAG GTA TTG AAC GCT ATT GAT GTG CTT TCC TAT ACC 450
136 Ala Tyr Phe Lys Val Leu Asn Ala Ile Asp Val Leu Ser Tyr Thr 150

451 CAG GCG CAA AAA GAG GCT ATC TAC CGT CAG TTA GAT CAA ACG ACG 495
151 Gln Ala Gln Lys Glu Ala Ile Tyr Arg Gln Leu Asp Gln Thr Thr 165

496 CAA CGT TTT AAC GTG GGT CTG GTC GCC ATT ACC GAC GTG CAA AAC 540
166 Gln Arg Phe Asn Val Gly Leu Val Ala Ile Thr Asp Val Gln Asn 180

541 GCC CGT GCG CAA TAT GAT ACC GTA CTG GCG AAT GAA GTG ACC GCC 585
181 Ala Arg Ala Gln Tyr Asp Thr Val Leu Ala Asn Glu Val Thr Ala 195

586 CGC AAC AAC CTG GAT AAC GCG GTA GAA GAG CTG CGC CAG GTA ACC 630
196 Arg Asn Asn Leu Asp Asn Ala Val Glu Glu Leu Arg Gln Val Thr 210

631 GGC AAT TAT TAC CCG GAG CTG GCG TCG CTT AAC GTC GAG CAT TTT 675
211 Gly Asn Tyr Tyr Pro Glu Leu Ala Ser Leu Asn Val Glu His Phe 225

676 AAA ACC GAC AAA CCC AAA GCT GTT AAT GCG CTG TTG AAG GAA GCG 720
226 Lys Thr Asp Lys Pro Lys Ala Val Asn Ala Leu Leu Lys Glu Ala 240

721 GAA AAC CGT AAC CTG TCG CTG TTG CAG GCG CGT TTA AGT CAG GAT 765

241 Glu Asn Arg Asn Leu Ser Leu Leu Gln Ala Arg Leu Ser Gln Asp 255

766 CTG GCG CGC GAG CAA ATC CGT CAG GCG CAG GAT GGT CAC CTG CCG 810
256 Leu Ala Arg Glu Gln Ile Arg Gln Ala Gln Asp Gly His Leu Pro 270

811 ACG CTG AAT TTA ACG GCC TCA ACC GGC ATT TCT GAT ACC TCT TAT 855
271 Thr Leu Asn Leu Thr Ala Ser Thr Gly Ile Ser Asp Thr Ser Tyr 285

856 AGC GGT TCT AAA ACC AAC TCC ACC CAG TAC GAC GAT AGC AAC ATG 900
286 Ser Gly Ser Lys Thr Asn Ser Thr Gln Tyr Asp Asp Ser Asn Met 300

901 GGG CAG AAT AAA ATC GGC CTT AAC TTC TCC CTG CCG CTG TAT CAA 945
301 Gly Gln Asn Lys Ile Gly Leu Asn Phe Ser Leu Pro Leu Tyr Gln 315

946 GGT GGG ATG GTT AAC TCG CAG GTA AAA CAG GCG CAG TAT AAC TTC 990
316 Gly Gly Met Val Asn Ser Gln Val Lys Gln Ala Gln Tyr Asn Phe 330

991 GTC GGC GCA AGC GAA CAG CTG GAA AGC GCG CAC CGT AGC GTG GTG 1035
331 Val Gly Ala Ser Glu Gln Leu Glu Ser Ala His Arg Ser Val Val 345

1036 CAG ACC GTA CGT TCT TCC TTT AAC AAT ATT AAC GCC TCC ATC AGC 1080
346 Gln Thr Val Arg Ser Ser Phe Asn Asn Ile Asn Ala Ser Ile Ser 360

1081 AGC ATC AAC GCG TAT AAA CAG GCG GTC GTT TCC GCG CAA AGT TCT 1125
361 Ser Ile Asn Ala Tyr Lys Gln Ala Val Val Ser Ala Gln Ser Ser 375

1126 TTG GAT GCC ATG GAA GCC GGT TAC TCG GTC GGT ACA CGT ACC ATT 1170
376 Leu Asp Ala Met Glu Ala Gly Tyr Ser Val Gly Thr Arg Thr Ile 390

1171 GTT GAC GTA CTG GAT GCC ACC ACC ACT CTG TAT GAT GCC AAG CAG 1215
391 Val Asp Val Leu Asp Ala Thr Thr Thr Leu Tyr Asp Ala Lys Gln 405

1216 CAA CTG GCC AAC GCG CGT TAT ACC TAT TTG ATT AAT CAG TTA AAT 1260
406 Gln Leu Ala Asn Ala Arg Tyr Thr Tyr Leu Ile Asn Gln Leu Asn 420

1261 ATC AAA TAT GCG CTC GGT ACG CTG AAC GAG CAG GAT CTG CTC GCG 1305
421 Ile Lys Tyr Ala Leu Gly Thr Leu Asn Glu Gln Asp Leu Leu Ala 435

1306 CTT AAC AGT ACG TTG GGT AAA CCT ATC CCG ACG TCG CCG GAA AGC 1350
436 Leu Asn Ser Thr Leu Gly Lys Pro Ile Pro Thr Ser Pro Glu Ser 450

1351 GTA GCG CCG GAA ACG CCA GAT CAG GAT GCT GCC GCA GAC GGT TAT 1395
451 Val Ala Pro Glu Thr Pro Asp Gln Asp Ala Ala Ala Asp Gly Tyr 465

1396 AAT GCT CAT AGC GCC GCG CCA GCA GTA CAG CCG ACC GCC GCT CGC 1440
466 Asn Ala His Ser Ala Ala Pro Ala Val Gln Pro Thr Ala Ala Arg 480

1441 GCC AAC AGC AAT AAC GGC AAT CCA TTC CGG CAT TGA 1476
481 Ala Asn Ser Asn Asn Gly Asn Pro Phe Arg His End 491

The invention has specifically contemplated each and every possible variation of polynucleotide that could be made by selecting combinations based on the possible codon choices listed in Table 1 and Table 2, and all such variations are to be considered as being specifically disclosed. Codons are preferably selected to fit the host cell in which the protein is being produced. Selection of codons to maximize expression of proteins in a heterologous host is a known technique.

### Table 2

### Genetic Code

The amino acids and their three letter and single letter abbreviations are mentioned inside the parenthesis. The codons are 3-letter triplets each representing a trinucleotide of DNA having a 5' end on the left and a 3' end on the right. The RNA code is the same except that U (uracil) replaces T

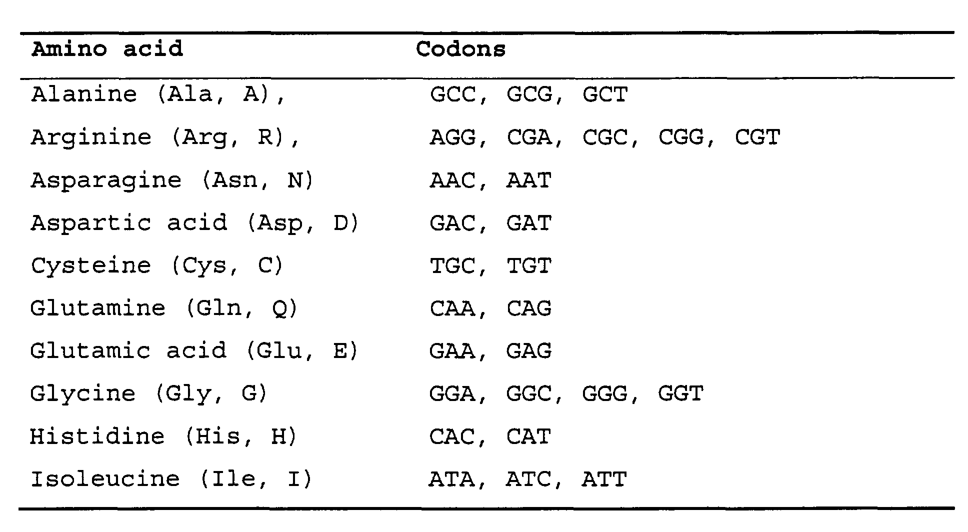

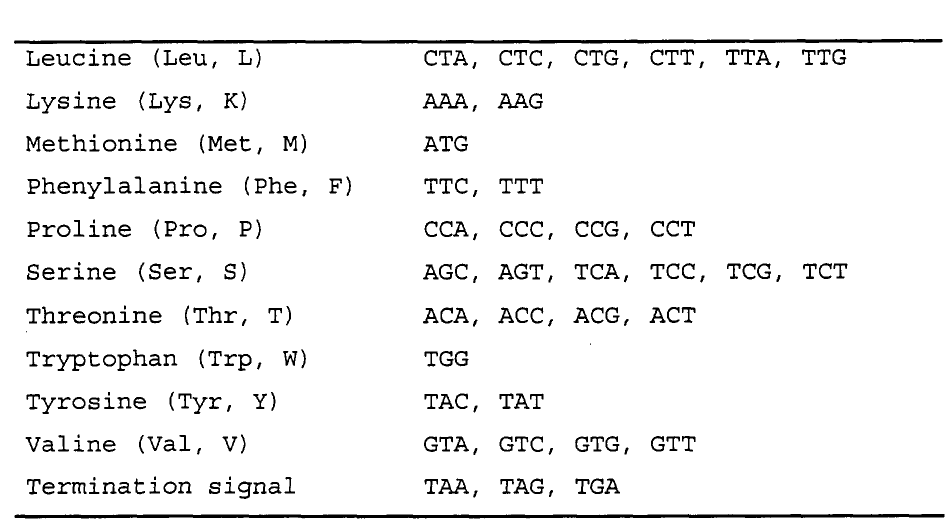

Other DNA molecules that code for such peptides can readily be determined from the list of codons in Table 2 and are likewise contemplated as being equivalent to the DNA sequence of Table 1. In fact since there is a fixed relationship between DNA codons and amino acids in a peptide, any discussion in this application of a replacement or other change in a peptide is equally applicable to the corresponding DNA sequence or to the DNA molecule, recombinant vector, or transformed microorganism in which the sequence is located (and vice versa).

In addition the specific nucleotides listed in Table 1, DNA (or corresponding RNA) molecules of the invention can have additional nucleotides preceding or following those that are specifically listed. For example, a short (e.g. fewer than 20 nucleotides) sequence can be added to the 3' terminal to provide a terminal sequence corresponding to a restriction endonuclease site, stop codons can follow the peptide sequence to terminate translation, and the like. Additionally, DNA molecules containing a promoter region or other control region upstream from the gene can also be produced. All DNA molecules containing the sequences of the invention will be useful for at least one purpose since all can minimally be fragmented to produce oligonucleotide probes and be used in the isolation or detection of DNA from biological sources.

A number of words used in this specification have specific means in addition to their more common meanings. By "equivalent" is meant, when referring to two nucleotide sequences, that the two nucleotide sequences in question encode the same sequence of amino acids. When "equivalent" is used in referring to two peptides, it means that the two peptides will have a common property (such as antigenic activity, as established by the context). The property does not need to be present to the same extent in both peptides (e.g. two peptides can exhibit different antigenic reactivity), but the properties are preferably substantially the same. "Complementary", when referring to two nucleotide sequences, means that the two sequences are capable of hybridizing, preferably with less than 25%, more preferably with less than 15%, even more preferably with less than 5%, most preferably with no mismatches between opposed nucleotides. Preferred hybridizing conditions (which are not limited to specific numbers of mismatches) are set forth in the Examples. The term "substantially" varies with the context as understood by those skilled in the relevant art and generally means at least 70%, preferably means at least 80%, more preferably at least 90%, and most preferably at least 95%. The term "isolated" as used herein refers to peptide, DNA, or RNA separated from other peptides, DNAs or RNAs, respectively, and being found in the presence of (if anything) only a solvent, buffer, ion or other component normally present in a biochemical solution of the same. "Isolated' does not encompass either natural materials in their native state or natural materials that have been separated into components (e.g. in an acrylamide gel) but not obtained either as pure substances or as solutions.

Since the DNA sequence of the gene has been identified, it is possible to produce a DNA gene entirely by synthetic chemistry, after which the gene can be inserted into any of the many available DNA vectors using known techniques of recombinant DNA technology. Thus, the present invention can be carried out using reagents, plasmids, and microorganisms which are freely available and in the public domain at the time of filing of this patent application without requiring a deposit of genetic material.

For example, the entire 1476bp of ST50 gene can be synthesized in a single reaction using specific oligonucleotides of 40 bases spanning both the strands of the gene. This can be done by "assembly PCR technique" that has been described in detail in, for example, Stemmer et al 1995, Gene 164:49-53.

The peptides can then be expressed in a host organism as described herein. Furthermore, automated equipment is also available that makes direct synthesis of many of the peptides disclosed herein readily available, especially peptide fragments of less that the entire 50kDa OMP of *S.typhi*. Such equipment provides ready access to the peptides of the invention, either by direct synthesis or by synthesis of a series of fragments that can be coupled using other known techniques.

In addition to the specific polypeptide sequence shown in Table 1, peptide fragments based on this sequence and fragments and full length sequences representing minor variations thereof will have some of the biological activities of the specific 50kDa OMP of *S.typhi* and will therefore be useful in vaccine or diagnostic development or other studies. For example, fragments of the 50kDa OMP sequence can be readily be prepared and can be screened for use as epitope for diagnostic or for DNA vaccine development. Peptide synthesizers can be used to prepare small polypeptide fragments (e.g., less than 100 amino acids) or techniques of genetic engineering can be used to prepare larger fragments. By antibody affinity chromatography using anti-50kDa OMP antibody immunogenic peptides/epitopes can be derived. Such peptides can also be used (and are indeed more likely to be used) as immunogens for the preparation of antibodies or as standards in assays that use antibodies to the *S.typhi* 50kDa OMP as a method of identifying the presence of a typhoid infection.

The ability to prepare and select peptide fragments having appropriate immunological reactivity from a larger protein is a well known art as described in a number of publications, including patents. For example, U.S. Pat. No. 4, 629, 783, which describes the preparation of immunologically active fragments of viral proteins that bind with the same antibodies as the entire viral protein.

In addition, minor variations of the previously mentioned peptides and DNA molecules are also contemplated as being equivalent to those peptides and DNA molecules that are set forth in more detail, as will be appreciated by those skilled in the art. For example, it is reasonable to expect that an isolated replacement of a leucine with an isoleucine or valine, an aspartate with a glutamate, a threonine with a serine, or a similar replacement of an amino acid with a structurally related amino acid (i.e., a conservative replacement) will not have a major effect on the biological activity of the resulting molecule, especially if the replacement does not involve an amino acid at a binding site or other site of biologic activity. This is particularly true of the ST50 gene in view of the known significant variations that exist between species. Furthermore, additional amino acids can be present at either of the two termini, or amino acids can be absent, from one or both of the termini, as is known in the art. Peptides in which more than one replacement has taken place can readily be tested in the same manner. Preferred peptides differ at no more than 12, more preferably no more than 5, amino acids in any contiguous group of 20 amino acids. Substitutions of amino acids, when they occur are preferably from within standard conservative groups. Standard conservative groups of amino acids are shown in parenthesis using the one-letter amino acid code (Table 2): nonpolar (A.V, L, I, P, M); aromatic (F, T, W); uncharged polar (G, S, T, C, N, Q); acidic (D,E); basic (K, R,H). The aromatic amino acids are sometimes considered to belong to the broader-defined nonpolar (F, W) or uncharged polar (T) groups. However, such modified protein is less useful in the specific diagnostic assays related to identification of *S.typhi.*

Salts of any of the peptides described herein will naturally occur when such peptides are present in (or isolated from) aqueous solutions of various pHs. All salts of peptides having the indicated biological activity are considered to be within the scope of the present invention. Examples include alkali, alkaline earth, and other metal salts of carboxylic acid residues, acid addition salts (e.g., HCI) of amino residues and zwitter ions formed by reactions between carboxylic acid and amino residues within the same molecule.

Peptides of the invention can be prepared for the first time as homogeneous preparations free of other Salmonella materials, either by direct synthesis or by using a cloned gene or a fragment thereof as described herein. *S.typhi* 50kDa OMP was previously available in the form of a crude homogenate with a purity of less than 0.1%. The crude preparation was not free of all other S. *typhi* materials. Although genes and corresponding proteins can be prepared by the totally synthetic techniques discussed above, in preferred embodiments of the invention genetic information is obtained from natural sources and identified as described herein. The genetic material is first obtained in the form of a gene library, using any of numerous existing techniques. The first of these is to randomly shear genomic DNA and insert this sheared material into expression vectors. If enough recombinants are generated, there is a good probability of having at least one recombinant in the population, which is expressing the 50kDa OMP protein.

Details of this example are set forth below, including details of the experiments that lead to obtaining the complete sequence of the gene. However, there is no reason to believe that the sequence and specific engineered organism prepared by the inventors is any better than other clones that can be prepared using the guidance set forth in this specification. In fact, it is likely that expression of the 50kDa OMP protein can be enhanced over that described herein by selection of other expression systems.

Now that the sequence of the ST50 gene has been determined, it is no longer necessary to go through these steps to obtain the genetic materials of the present invention. The polymerase chain reaction (PCR) technique can now be used to isolate genes from natural sources in a simpler and more direct manner. The PCR technique, including its use in diagnosis, is disclosed in U.S. patent: 4, 683, 202, which is, herein incorporated by reference. Since *S.typhi* specimens are readily available from sources such as the American Type Culture Collection of Rockville, Md., and since PCR probes can be prepared using the sequences set forth in this specification, it is possible to obtain any desired segment of the sequences set forth herein using the PCR technique and commercially available sources of the *S.typhi* genomic material. A specific example of such a technique for isolating the *S.typhi* chromosomal gene is described in the examples that follow.

Although the techniques set forth above, when used in combination with the knowledge of those skilled in the art of genetic engineering and the previously stated guidelines, will readily enable isolation of the desired gene and its use in recombinant DNA vectors now that sufficient information is provided to locate the gene, other methods which lead to the same result are also known and may be used in the preparation of recombinant DNA vectors of this invention.

Expression of 50kDa OMP can be enhanced by including multiple copies of the gene in a transformed host; by selecting a vector known to reproduce in the host, thereby producing large quantities of the said protein from exogenous inserted DNA; or by any other known means of enhancing peptide expression. One common variation is the preparation of a polypeptide of the invention in the form of a fused polypeptide. Such peptides are typically prepared by using a plasmid vector with a promoter region of a gene known to be expressed and inserting nucleotides that encode all or a major portion of the amino acid sequence of the invention into the genetic sequence. Examples of such fused proteins include histidine-tag, beta-galactosidase, maltose binding protein and green fluorescent protein. If desired, the fused peptide can be designed so that a site recognized by a proteolytic enzyme (example: enterokinase) is present at the junction between the two fused proteins. The proteolytic enzyme can then be used to cleave the expressed protein so that the desired *S.typhi* specific 50kDa OMP is available in pure form.

In all cases, S. *typhi* 50kDa OMP protein will be expressed when the DNA sequence is functionally inserted into the vector. By "functionally inserted" is meant in proper reading frame and orientation, as is well understood by those skilled in the art. Typically, a gene will be inserted downstream from a promoter and will be followed by a stop codon, although production as a hybrid protein (possibly followed by cleavage) may be used, if desired.

In addition to the above general procedures which can be used for preparing recombinant DNA molecules and transformed in unicellular organisms in accordance with the practices of this invention, other known techniques and modifications thereof can be used in carrying out the practice of the invention. In particular, techniques relating to genetic engineering have recently undergone explosive growth and development. Many recent US patents disclose plasmids, genetically engineering microorganisms, and methods of conducting genetic engineering, which can be used in the practice of the present invention. For example, U.S. Patent No. 4, 273, 875 discloses a plasmid and a process of isolating the same. U.S. Patent No. 4, 304, 863 discloses a process for producing bacteria by genetic engineering in which a hybrid plasmid is constructed and used to transform a bacterial host, U.S. Patent No. 4, 419, 450 discloses a plasmid useful as a cloning vehicle in recombinant DNA work. U.S. Patent No. 4, 362, 867 discloses recombinant cDNA construction methods and hybrid nucleotides produced thereby which are useful in cloning processes. U.S. Patent No. 4, 403, 036 discloses genetic reagents for generating plasmids containing multiple copies of DNA segments. U.S. Patent No. 4, 363, 877 discloses recombinant DNA transfer vectors. U.S. Patent No. 4, 356,270 discloses a recombinant DNA cloning vehicle and is a particularly useful disclosure for those with limited experience in the area of genetic engineering since it defines many of the terms used in genetic engineering and the basic processes used therein. U.S. Patent No. 4, 336, 336 discloses a fused gene and a method of making the same. U.S. Patent No. 4, 349, 629 discloses plasmid vectors and the production and use thereof. U.S.Patent No.4, 332,901 discloses a cloning vector useful in recombinant DNA. Although some of these patents are directed to the production of a particular gene product that is not within the scope of the present invention, the procedures described therein can easily be modified to the practice of the invention described in this specification by those skilled in the art of genetic engineering.

The implications of the present invention are significant in that useful amounts of *S*. *typhi* specific 50kDa OMP and genetic material of the invention will become available for use in the development of hybridization assays or in any other type of assay utilizing these materials as a reagent for use in diagnosis, immunization, therapeutics, and research. Transferring the ST50 gene or parts thereof which has been isolated to other expression vectors will produce constructs which improve the expression of the *S*. *typhi* specific 50kDa OMP in *E. coli* or express the polypeptide in other hosts.

Particularly contemplated is the isolation of genes from other strains of *S.typhi* using oligonucleotide probes based on the principal and variant nucleotide sequences disclosed herein. Such probes can be considerably shorter than the entire sequence but should be at least 10, preferable at least 14, nucleotides in length. Intermediate oligonucleotides from 20 to 500, especially 30 to 200, nucleotides in length provide particularly specific and rapid-acting probes. Longer oligonucleotides are also useful, up to the full length of the gene. Both RNA and DNA probes can be used.

In use, the probes are typically labelled in a detectable manner (e.g. P³², S³⁵, biotin, avidin, fluorescein or digoxigenin) and are incubated with single-stranded DNA or RNA from the organism in which a gene is being sought. Hybridization is detected by means of the label denaturing the double-stranded (hybridized) DNA (or DNA/RNA) have been separated (typically using nitrocellulose paper). Hybridization techniques suitable for use with oligonucleotides are well known.

Although probes are normally used with a detectable label that allows easy identification, unlabelled oligonucleotides are also useful, both as precursors of labelled probes and for use in methods that provide for direct detection of double-stranded DNA (or DNA/RNA). Accordingly, the term "oligonucleotide probe" refers to both labelled and unlabelled forms.

Monoclonal or polyclonal antibodies to recombinant ST50 protein can be produced using the known technologies. Antibodies produced against the ST50 recombinant protein could be used to develop an antigen detection immuno assay for the detection of S. typhi or for use with other technologies which use antibody as an ingredient. In addition, parts of the DNA or the peptides or proteins derived from the ST50 gene can be used as an immunogen in vaccine formulations to protect against S. typhi infections. The immunogen may be incorporated into liposomes, or conjugated to polysaccharides and/or other polymers for use in a vaccine formulation. The DNA sequence of ST50 gene can also be used to develop a DNA vaccine formulation. Many methods may be used by those skilled in the art, to introduce the vaccine formulations into human. These include, but are not limited to intradermal, intramuscular, intraperitoneal, intravenous, subcutaneous, oral and intranasal routes of administration.

In summary, *S.typhi* specific ST50 gene have been isolated, cloned, sequenced and expressed in *E.coli*. The open-reading frame of this gene directs the synthesis of a 50,000 Dalton protein with identifiable similarities to the 50kDa OMP of S.*typhi*. The sequence of the entire ST50 gene was 1476 base pairs (bp) with GC and AT composition of 52.03% and 47.97% respectively. The open reading frame (ORF) starts at 7bp nucleotide position with a start codon "ATG" and a stop codon "TGA" at 1474bp nucleotide position. The predicted amino acid of the ST50 gene consisted of 491 amino acids. The signal sequence of the 50kDa OMP is a 16 amino acid peptide that extends between the 3rd and 18^{th} amino acid position. The 50kDa OMP has been expressed in large quantities in *E.coli*, which provides a source for large quantities of pure protein for future diagnostic and vaccine studies.

The expressed 50kDa OMP has been purified to the homogeneity by IMAC chromatography. The specific immuno reactivity of the recombinant ST50 protein has been evaluated by Western blot and Dot immuno assay at IgM and IgG level. With the availability of information about the DNA sequence, it should not be difficult to use the same or parts of it as DNA probe or PCR primer for the diagnosis of typhoid. This invention now being generally described, the same will be better understood by reference to the following examples, which are provided for purposes of illustration only and are not to be considered limiting of the invention unless so specified.

### Examples

### Bacterial strains and Media

Salmonella typhi (USM1) strain was isolated from a patient with typhoid fever and has been maintained in our laboratory since 1987. A sample of this bacterial strain has been deposited at the Australian Government, Analytical Laboratories, P O Box 385, Pymble, NSW 2073, Australia, under Accession Number NM00/16074. Blood agar and nutrient broth was used to propagate the S. typhi strain. For molecular biology experiments, the S. typhi was cultured on Luria broth.

### Isolation of outer membrane protein

Partially purified OMP of *S. typhi* were obtained as described in Schnaitman *et al* (1971), 108: 553-556. Briefly *S. typhi* was grown in nutrient broth and incubated in a shaker at 37°C for 18 h until late log phase (OD =0.8 at 660nm). Cells were harvested and suspended in 0.01M HEPES buffer (pH 7.4). Bacterial cells were disrupted by vortexing with glass beads (0.15mm diameter) for 1.5h with 1 minute alternate on ice until 95% breakage was obtained as monitored by serial Gram stain. The cell lysate was aspirated and the glass beads washed with 0.01M HEPES buffer. Cell debris and unbroken cells were removed by centrifugation at 5,000g for 15min at 4°C. The supernatant fluid was centrifuged at 200,000g for 1h to obtain cell envelopes. The cytoplasmic membrane was removed by 0.01M HEPES containing 2% Triton X-100 and was allowed to stand for 10 min at room temperature. This was followed by centrifugation at 200,000g for 1h, 4°C to pellet the insoluble outer membrane. Protein concentration of OMP was determined by the colorimetric microassay using the Bio-Rad protein assay dye reagent (Bio-Rad, Richmond, CA) using bovine serum albumin as standard.

### Purification of 50kDa OMP from S. typhi

The 50kDa OMP of *S*. *typhi* was isolated using sodium dodecyl sulfate- polyacrylamide gel electrophoresis (SDS-PAGE) and electro elution. SDS-PAGE was performed under reducing conditions using the discontinuous buffer systems (Lammli, 1970) with a vertical slab electrophoresis unit (Bio-Rad). The stacking and separating gels contained 4.5% and 9% acrylamide respectively. Each preparative gel was loaded with 500µg of OMP and was run at constant current setting of 25mA per plate at 4°C for 4 h. The separated OMPs were stained with Coomassie blue, and the molecular weights were established with molecular weight markers of 14.4 - 94kDa.

For N terminal amino acid sequencing the OMP was run on SDS-PAGE and electroblotted onto Polyvinylidene difluoride (PVDF) membrane. After staining with Coomassie blue, the 50kDa band of interest was excised carefully and sent for amino acid sequencing (Midwest Analytical, USA).

### Cloning of ST50 gene.

### (i) Primer designing

Amino acid sequence obtained from the N terminal amino acid sequencing of the ST50 OMP was analysed. Based on the analysis, a pair of PCR primers

STPCS1-ATG CAA ATG AAG AAA TTG CTC and STR1-TCA ATG CCG GAA TGG ATT GC were designed to PCR amplify the ST50 gene.

### (ii) Polymerase chain reaction (PCR)

*S*. *typhi* (USM1) genomic DNA was extracted by standard protocol and the 50ng of the DNA was used as a template for the PCR amplification using STPS1 and STR1. PCR amplification was performed under following conditions. 200 µM of each dNTP, 1X PCR buffer (50 mM KCl, 10 mM Tris.Cl, pH 8.3), 2.5 mM MgCl₂, 20pmol of STPCS1 and STR1 primer, and 1 unit of TAQ DNA polymerase. PCR was performed on a Perkin-Elmer 9600 Thermal cycler. The optimal annealing temperature of 50°C was used for the primer sets. The PCR parameters used are as follows: Initial denaturation at 95oC for 5 minutes, followed by additional 1 minute. Primer annealing and extension was carried out at 55°C and 72°C respectively for 1 minute. The steps were repeated for 30 cycles. Finally a temperature of 72°C was included for 5 minutes as a final extension. The PCR products were analyzed by agarose gel electrophoresis and the size of the PCR product was 1476bp. The PCR products were specific for *S*. *typhi* since the primers did not amplify any products when *E.coli* genomic DNA was used as template.

### (iii) PCR cloning of ST50 gene.

The amplified 1476bp PCR product of the ST50 gene was cloned on to PCR cloning vector (TOPO 2.1, Invitrogen) as per the manufacturer instructions. The clones were screened using an internal primer STPR1:GGC CGT TAA ATT CAG CGT CG and M13 reverse primer: CAG GAA ACA GCT ATG AC. The map of the ST50 clone pST50-USM1 is depicted in (Figure-1)

### Sequence of ST50 gene

The DNA sequencing of the ST50 gene was carried out using pST50-USM1 and M13 -20 forward CTG GCC GTC GTT TTA C and M13 reverse primers CAG GAA ACA GCT ATG AC through a commercial DNA sequencing company (ACGT Inc, USA). The sequence of the entire ST50 gene was 1476base pairs (bp) with GC and AT composition of 52.03% and 47.97%. The open reading frame (ORF) starts at 7bp nucleotide position with a start codon "ATG" and with a stop codon "TGA" at 1474bp nucleotide position. The ST50 gene code for 491 amino acids and the predicted size of the protein is 53682 Daltons. The signal sequence of the ST50 protein is a 16 amino acid peptide that extends between 3^{rd} and 18^{th} amino acid position. The DNA sequence and the predicted amino acid sequence are given in Table 1. The presence of signal sequence indicates that this protein is localized in the periplasmic region.

### Cloning of ST50 gene in expression vector

For the expression of ST50 gene, we choose a T7 promoter based expression vector system, pRSETB vector (Invitrogen), The advantage of pRSETB vector is that it contains T7 promoter, which is highly specific for T7 RNA Polymerase. Transcription by T7 polymerase is selective and 5 times faster than *E. coli* RNA polymerase thus leading to higher expression of genes cloned under T7 promoter. This vector also contains a nucleotide sequence that encodes a metal binding domain, a series of six consecutive histidine amino acids expressed as N-terminal fusion to the protein of interest. This metal binding domain (six-tagged histidine moieties) on the fusion peptide has high affinity for the divalent ions (like nickel, copper and cobalt) and facilitates one step purification of the protein using (IMAC) immobilized metal affinity columns.

From the TOPO vector the ST50 gene was excised using *Eco*RI restriction enzyme. The excised ST50kDa gene was ligated on to EcoRI site of pRSETB vector. The clones were screened using an internal primer STPR1: GGC CGT TAA ATT CAG CGT CG and T7 terminator primer: GCT AGT TAT TGC TCA GCG G. The map of the ST50 gene in pRSETB vector, pST50-USM2, is depicted in (Figure-2). The presence of ST50 gene in pRSETB vector was confirmed by restriction analysis using *Eco* RI restriction enzyme.

### Protein expression

The protein expression of ST50 protein from the pST50-USM2 clone was carried out in BL21(DE3) E. coli host. The E.coli BL21(DE3) contains chromosomal copy of T7 RNA polymerase gene under the control of lac UV5 promoter and hence expression of genes cloned under T7 promoter can be induced with gratuitous inducer such as IPTG. Further BL21(DE3) being a lon protease deficient strain which protects the expressed heterologous proteins from proteolytic cleavage. The ST50 protein was expressed in E. coli BL21(DE3) after inducing with IPTG.

Briefly the following protocol was followed for expression of the recombinant proteins:(Luria broth was supplemented with 100 µg/ml ampicillin for the following experiment)
a) E. coli BL21(DE3) was transformed with the pST50-USM2 clone using protocol mentioned in ' (Sambrook et al 1989) Molecular Cloning - A Laboratory Manual (second edition)', Cold Spring Harbor Laboratory, Cold Spring Harbor, New York.
b) A single colony of fresh transformant was inoculated into 1.5 ml LB and grown overnight (o/n) at 37oC, at static condition.
c) 50 µl of the o/n culture was inoculated into 10 ml LB in 100 ml conical flask and grown at 37oC with 150 rpm shaking, till OD600 of the culture reached 0.6.
d) IPTG was added to a final concentration of 1 mM and the culture was grown for 3 hours at 37oC with 150 rpm shaking.
e) The culture was centrifuged at 10,000g for 5 minutes. The supernatant was discarded and E. coli pellet containing the recombinant protein was stored at -20oC.

The recombinant ST50 protein was analysed by SDS-PAGE. The size of recombinant ST50 protein was 59kDa, that is larger than native 50kDa OMP, because of the presence of additional 52 amino acids derived from the pRSETB vector.

### Purification of the recombinant ST50 protein

The purification of recombinant ST50 protein was carried out by Immobilized metal affinity chromatography (IMAC). IMAC is a special form of affinity chromatography in which an immobilized metal ion such as copper, zinc or a transition metal ion such as cobalt or nickel is used to bind protein selectively by reaction with imidazole group of histidine residues. (Porath et al., 1975). The elution of the protein can be achieved either by lowering the pH, thereby destabilizing the protein-metal complex or by using competitive ligands like imidazole or by using complexing agents like EDTA.

In the clone pST50-USM2, the recombinant ST50 protein was expressed in the form of inclusion bodies. Therefore the recombinant ST50 proteins was solubilized with 8M urea and the protein was purified under denaturing conditions.

The following chromatography protocol was followed to purify the recombinant ST50 protein:

The NiNTA matrix was purchased from (Qiagen) and used for the purification of the recombinant ST50 protein. The column was prepared as described by the manufacture's instructions. Initially the matrix was equilibriated with the starting column buffer (0.1M phosphate buffer pH 8.0, 0.01M Tris pH 8.0 with 8M urea). The recombinant ST50 protein was solubilized by using lysis buffer (0.1M phosphate buffer pH 8.0, 0.01M Tris pH 8.0 and 8M Urea) for 8 hours at 4°C and spun at 12K for 15minutes to remove the debris. The supernatant containing the solubilized recombinant protein was passed through the column and allowed to bind for 4 hours. The unbound protein was removed by washing the column with 5 to 10 columns volumes of start buffer (0.1M phosphate buffer pH 8.0 containing 8M urea) until the fractions showed zero at A₂₈₀. The elution of the protein was achieved by 0.1M phosphate buffer pH 4.5 containing 8M Urea. The eluted fractions were collected and the protein concentration quantitated by measuring the absorbance at 280nm. The eluted fractions containing the protein were pooled and urea was removed by stepwise dialysis. SDS-PAGE analysis was carried out with the wash and the eluted fractions to check the purity of the eluted protein

### Immunoreactivity of recombinant ST50 protein

### (i) Western blotting

The immuno reactivity of the recombinant ST50 protein was analysed by western blotting. Briefly, the recombinant ST50 protein was run on a 8% SDS-PAGE. After electrophoresis the gel was incubated for 10 minutes in transfer buffer (25 mM Tris, 192mM glycine, 20% methanol). Nitrocellulose membrane (NCP) cut to the exact size of separating gel was incubated for 10 minutes in transfer buffer. Without trapping air-bubbles the NCP was overlaid on the gel and sandwiched between filter papers and scotch brite pads. Electrophoretic transfer was carried out in the cold room at 200 mA for 3 hours using Transblot (Bio Rad,USA) electroblotting apparatus. After transfer, the molecular weight marker lane was cut and stained with amido black (100 mg Amido block in 45% methanol, 10% acetic acid). The rest of the NCP was stained with Ponceau S (0.2% Ponceau S, Sigma, USA, in 0.3% trichloro acetic acid and 0.3% sulfosalicylic acid) to ensure the transfer of the proteins. Membrane was washed in PBS and blocked o/n at 4°C with 5% non-fat milk powder in PBS.The NCP was washed in wash buffer (PBS) thrice 5 minutes duration each, and then incubated over night with 1:200 dilution of pooled normal sera, pooled typhoid sera, sera from patients with fevers common in this region such as dengue, hepatitis, scrub typhus, paratyphi A, B and C. The pooled normal sera was obtained from 5 healthy individuals while pooled typhoid sera was obtained from typhoid patient who's blood samples were positive by culture. After washing in the wash buffer the membrane was incubated for 2 hours with peroxidase-conjugated antihuman IgM and IgG. After extensive washing the blot was developed using H₂O₂ and 4-chloro-1-naphthol reagent for 15 minutes and the blot was rinsed in distilled water to stop the reaction. The recombinant ST50 protein specifically reacted with *S*. *typhi* patients sera at the IgG and at the IgM antibody level but not with sera obtained from other infections.

### Dot enzyme immuno assay.

A nitrocellulose membrane of 0.45µ pore size (Microfiltration system, CA, USA) was used in this assay. One µl of the recombinant ST50 protein i.e., protein was dotted onto nitrocellulose using a microsyringe and allowed to dry. The strips were dipped into blocking buffer (3% skimmed milk, 0.9% NaCl, 10mM Tris-HCl, pH 7.4), and placed on a rocker platform for 30 minutes at room temperature. The blocked strips were rinsed 3 times for 15 minutes with NETG buffer (150 mM NaCl, 50mM Tris-HCl, 5mM EDTA and 0.25% gelatin), allowed to dry and kept at 4°C until use. The strip was then probed with one ml of 1:100 dilution of the different sera sample and incubated on a rocker platform for 1hour at room temperature. The strips were then washed with 3 times for 15 minutes with 1M NETG buffer and further incubated on a rocker platform with 1: 1,600 dilution of peroxidase conjugated antihuman IgG (Dakopatts, Glostrup, Denmark) or 1:800 dilution of antihuman IgM (Dakopatts, Glostrup, Denmark). After extensive washing the blot was developed using H₂O₂ and 4-chloro-1-naphthol reagent for 15 minutes and the blot was rinsed in distilled water to stop the reaction. The recombinant ST50 protein showed specific reactivity with *S. typhi* patients sera at the IgM and at the IgG antibody level whereas no reactivity was seen with sera obtained from other infections.

The isolated DNA or RNA molecule (ST50) of the invention are useful as sources of immunological protection against diseases such as typhoid fever in an animal, example, a mammal, example, a human, in particular as the basis of a vaccine capable of provoking a strong immune reaction.

Appropriate dosages and conditions of administrations are known in the art.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

Seq ID1 - Sequence Listings

1 ATG CAA ATG AAG AAA TTG CTC CCC ATC CTT ATC GGC CTG AGC CTG 45
1 Met Gln Met Lys Lys Leu Leu Pro Ile Leu Ile Gly Leu Ser Leu 15

46 TCG GGG TTC AGC ACA CTA AGC CAG GCA GAG AAC CTG ATG CAA GTT 90
16 Ser Gly Phe Ser Thr Leu Ser Gln Ala Glu Asn Leu Met Gln Val 30

91 TAT CAG CAA GCA CGC CTG AGC AAC CCG GAA TTG CGT AAA TCC GCT 135
31 Tyr Gln Gln Ala Arg Leu Ser Asn Pro Glu Leu Arg Lys Ser Ala 45

136 GCC GAT CGC GAT GCT GCA TTC GAA AAA ATT AAC GAA GCG CGT AGT 180
46 Ala Asp Arg Asp Ala Ala Phe Glu Lys Ile Asn Glu Ala Arg Ser 60

181 CCT TTA CTG CCG CAA CTG GGT TTA GGT GCC GAC TAC ACC TAC AGC 225
61 Pro Leu Leu Pro Gln Leu Gly Leu Gly Ala Asp Tyr Thr Tyr Ser 75

226 AAC GGT TAT CGC GAT GCG AAC GGT ATC AAC TCC AAT GAA ACC AGC 270
76 Asn Gly Tyr Arg Asp Ala Asn Gly Ile Asn Ser Asn Glu Thr Ser 90

271 GCT TCT CTG CAA TTA ACG CAG ACG CTA TTT GAT ATG TCG AAA TGG 315
91 Ala Ser Leu Gln Leu Thr Gln Thr Leu Phe Asp Met Ser Lys Trp 105

316 CGT GGG CTC ACC CTG CAA GAA AAA GCA GCA GGC ATT CAG GAT GTC 360
106 Arg Gly Leu Thr Leu Gln Glu Lys Ala Ala Gly Ile Gln Asp Val 120

361 ACC TAT CAG ACC GAT CAG CAG ACG CTG ATC CTC AAT ACC GCG AAC 405
121 Thr Tyr Gln Thr Asp Gln Gln Thr Leu Ile Leu Asn Thr Ala Asn 135

406 GCG TAT TTT AAG GTA TTG AAC GCT ATT GAT GTG CTT TCC TAT ACC 450
136 Ala Tyr Phe Lys Val Leu Asn Ala Ile Asp Val Leu Ser Tyr Thr 150

451 CAG GCG CAA AAA GAG GCT ATC TAC CGT CAG TTA GAT CAA ACG ACG 495
151 Gln Ala Gln Lys Glu Ala Ile Tyr Arg Gln Leu Asp Gln Thr Thr 165

496 CAA CGT TTT AAC GTG GGT CTG GTC GCC ATT ACC GAC GTG CAA AAC 540
166 Gln Arg Phe Asn Val Gly Leu Val Ala Ile Thr Asp Val Gln Asn 180

541 GCC CGT GCG CAA TAT GAT ACC GTA CTG GCG AAT GAA GTG ACC GCC 585
181 Ala Arg Ala Gln Tyr Asp Thr Val Leu Ala Asn Glu Val Thr Ala 195

586 CGC AAC AAC CTG GAT AAC GCG GTA GAA GAG CTG CGC CAG GTA ACC 630
196 Arg Asn Asn Leu Asp Asn Ala Val Glu Glu Leu Arg Gln Val Thr 210

631 GGC AAT TAT TAC CCG GAG CTG GCG TCG CTT AAC GTC GAG CAT TTT 675
211 Gly Asn Tyr Tyr Pro Glu Leu Ala Ser Leu Asn Val Glu His Phe 225

676 AAA ACC GAC AAA CCC AAA GCT GTT AAT GCG CTG TTG AAG GAA GCG 720

226 Lys Thr Asp Lys Pro Lys Ala Val Asn Ala Leu Leu Lys Glu Ala 240

721 GAA AAC CGT AAC CTG TCG CTG TTG CAG GCG CGT TTA AGT CAG GAT 765
241 Glu Asn Arg Asn Leu Ser Leu Leu Gln Ala Arg Leu Ser Gln Asp 255

766 CTG GCG CGC GAG CAA ATC CGT CAG GCG CAG GAT GGT CAC CTG CCG 810
256 Leu Ala Arg Glu Gln Ile Arg Gln Ala Gln Asp Gly His Leu Pro 270

811 ACG CTG AAT TTA ACG GCC TCA ACC GGC ATT TCT GAT ACC TCT TAT 855
271 Thr Leu Asn Leu Thr Ala Ser Thr Gly Ile Ser Asp Thr Ser Tyr 285

856 AGC GGT TCT AAA ACC AAC TCC ACC CAG TAC GAC GAT AGC AAC ATG 900
286 Ser Gly Ser Lys Thr Asn Ser Thr Gln Tyr Asp Asp Ser Asn Met 300

901 GGG CAG AAT AAA ATC GGC CTT AAC TTC TCC CTG CCG CTG TAT CAA 945
301 Gly Gln Asn Lys Ile Gly Leu Asn Phe Ser Leu Pro Leu Tyr Gln 315

946 GGT GGG ATG GTT AAC TCG CAG GTA AAA CAG GCG CAG TAT AAC TTC 990
316 Gly Gly Met Val Asn Ser Gln Val Lys Gln Ala Gln Tyr Asn Phe 330

991 GTC GGC GCA AGC GAA CAG CTG GAA AGC GCG CAC CGT AGC GTG GTG 1035
331 Val Gly Ala Ser Glu Gln Leu Glu Ser Ala His Arg Ser Val Val 345

1036 CAG ACC GTA CGT TCT TCC TTT AAC AAT ATT AAC GCC TCC ATC AGC 1080
346 Gln Thr Val Arg Ser Ser Phe Asn Asn Ile Asn Ala Ser Ile Ser 360

1081 AGC ATC AAC GCG TAT AAA CAG GCG GTC GTT TCC GCG CAA AGT TCT 1125
361 Ser Ile Asn Ala Tyr Lys Gln Ala Val Val Ser Ala Gln Ser Ser 375

1126 TTG GAT GCC ATG GAA GCC GGT TAC TCG GTC GGT ACA CGT ACC ATT 1170
376 Leu Asp Ala Met Glu Ala Gly Tyr Ser Val Gly Thr Arg Thr Ile 390

1171 GTT GAC GTA CTG GAT GCC ACC ACC ACT CTG TAT GAT GCC AAG CAG 1215
391 Val Asp Val Leu Asp Ala Thr Thr Thr Leu Tyr Asp Ala Lys Gln 405

1216 CAA CTG GCC AAC GCG CGT TAT ACC TAT TTG ATT AAT CAG TTA AAT 1260
406 Gln Leu Ala Asn Ala Arg Tyr Thr Tyr Leu Ile Asn Gln Leu Asn 420

1261 ATC AAA TAT GCG CTC GGT ACG CTG AAC GAG CAG GAT CTG CTC GCG 1305
421 Ile Lys Tyr Ala Leu Gly Thr Leu Asn Glu Gln Asp Leu Leu Ala 435

1306 CTT AAC AGT ACG TTG GGT AAA CCT ATC CCG ACG TCG CCG GAA AGC 1350
436 Leu Asn Ser Thr Leu Gly Lys Pro Ile Pro Thr Ser Pro Glu Ser 450

1351 GTA GCG CCG GAA ACG CCA GAT CAG GAT GCT GCC GCA GAC GGT TAT 1395
451 Val Ala Pro Glu Thr Pro Asp Gln Asp Ala Ala Ala Asp Gly Tyr 465

1396 AAT GCT CAT AGC GCC GCG CCA GCA GTA CAG CCG ACC GCC GCT CGC 1440
466 Asn Ala His Ser Ala Ala Pro Ala Val Gln Pro Thr Ala Ala Arg 480

1441 GCC AAC AGC AAT AAC GGC AAT CCA TTC CGG CAT TGA 1476
481 Ala Asn Ser Asn Asn Gly Asn Pro Phe Arg His End 491

## Claims

1. An isolated DNA or RNA molecule (ST50), which comprises a nucleotide sequence encoding for the specific OMP of Salmonella typhi which is estimated to have a molecular weight of 50kDa.

2. The molecule of claim 1, wherein said molecule comprises of the *S*. *typhi* specific OMP protein coding sequence: 
1 ATG CAA ATG AAG AAA TTG CTC CCC ATC CTT ATC GGC CTG AGC CTG 45
1 Met Gln Met Lys Lys Leu Leu Pro Ile Leu Ile Gly Leu Ser Leu 15

46 TCG GGG TTC AGC ACA CTA AGC CAG GCA GAG AAC CTG ATG CAA GTT 90
16 Ser Gly Phe Ser Thr Leu Ser Gln Ala Glu Asn Leu Met Gln Val 30

91 TAT CAG CAA GCA CGC CTG AGC AAC CCG GAA TTG CGT AAA TCC GCT 135
31 Tyr Gln Gln Ala Arg Leu Ser Asn Pro Glu Leu Arg Lys Ser Ala 45

136 GCC GAT CGC GAT GCT GCA TTC GAA AAA ATT AAC GAA GCG CGT AGT 180
46 Ala Asp Arg Asp Ala Ala Phe Glu Lys Ile Asn Glu Ala Arg Ser 60

181 CCT TTA CTG CCG CAA CTG GGT TTA GGT GCC GAC TAC ACC TAC AGC 225
61 Pro Leu Leu Pro Gln Leu Gly Leu Gly Ala Asp Tyr Thr Tyr Ser 75

226 AAC GGT TAT CGC GAT GCG AAC GGT ATC AAC TCC AAT GAA ACC AGC 270
76 Asn Gly Tyr Arg Asp Ala Asn Gly Ile Asn Ser Asn Glu Thr Ser 90

271 GCT TCT CTG CAA TTA ACG CAG ACG CTA TTT GAT ATG TCG AAA TGG 315
91 Ala Ser Leu Gln Leu Thr Gln Thr Leu Phe Asp Met Ser Lys Trp 105

316 CGT GGG CTC ACC CTG CAA GAA AAA GCA GCA GGC ATT CAG GAT GTC 360
106 Arg Gly Leu Thr Leu Gln Glu Lys Ala Ala Gly Ile Gln Asp Val 120

361 ACC TAT CAG ACC GAT CAG CAG ACG CTG ATC CTC AAT ACC GCG AAC 405
121 Thr Tyr Gln Thr Asp Gln Gln Thr Leu Ile Leu Asn Thr Ala Asn 135

406 GCG TAT TTT AAG GTA TTG AAC GCT ATT GAT GTG CTT TCC TAT ACC 450
136 Ala Tyr Phe Lys Val Leu Asn Ala Ile Asp Val Leu Ser Tyr Thr 150

451 CAG GCG CAA AAA GAG GCT ATC TAC CGT CAG TTA GAT CAA ACG ACG 495
151 Gln Ala Gln Lys Glu Ala Ile Tyr Arg Gln Leu Asp Gln Thr Thr 165
 
496 CAA CGT TTT AAC GTG GGT CTG GTC GCC ATT ACC GAC GTG CAA AAC 540
166 Gln Arg Phe Asn Val Gly Leu Val Ala Ile Thr Asp Val Gln Asn 180

541 GCC CGT GCG CAA TAT GAT ACC GTA CTG GCG AAT GAA GTG ACC GCC 585
181 Ala Arg Ala Gln Tyr Asp Thr Val Leu Ala Asn Glu Val Thr Ala 195

586 CGC AAC AAC CTG GAT AAC GCG GTA GAA GAG CTG CGC CAG GTA ACC 630
196 Arg Asn Asn Leu Asp Asn Ala Val Glu Glu Leu Arg Gln Val Thr 210

631 GGC AAT TAT TAC CCG GAG CTG GCG TCG CTT AAC GTC GAG CAT TTT 675
211 Gly Asn Tyr Tyr Pro Glu Leu Ala Ser Leu Asn Val Glu His Phe 225

676 AAA ACC GAC AAA CCC AAA GCT GTT AAT GCG CTG TTG AAG GAA GCG 720
226 Lys Thr Asp Lys Pro Lys Ala Val Asn Ala Leu Leu Lys Glu Ala 240

721 GAA AAC CGT AAC CTG TCG CTG TTG CAG GCG CGT TTA AGT CAG GAT 765
241 Glu Asn Arg Asn Leu Ser Leu Leu Gln Ala Arg Leu Ser Gln Asp 255

766 CTG GCG CGC GAG CAA ATC CGT CAG GCG CAG GAT GGT CAC CTG CCG 810
256 Leu Ala Arg Glu Gln Ile Arg Gln Ala Gln Asp Gly His Leu Pro 270

811 ACG CTG AAT TTA ACG GCC TCA ACC GGC ATT TCT GAT ACC TCT TAT 855
271 Thr Leu Asn Leu Thr Ala Ser Thr Gly Ile Ser Asp Thr Ser Tyr 285

856 AGC GGT TCT AAA ACC AAC TCC ACC CAG TAC GAC GAT AGC AAC ATG 900
286 Ser Gly Ser Lys Thr Asn Ser Thr Gln Tyr Asp Asp Ser Asn Met 300

901 GGG CAG AAT AAA ATC GGC CTT AAC TTC TCC CTG CCG CTG TAT CAA 945
301 Gly Gln Asn Lys Ile Gly Leu Asn Phe Ser Leu Pro Leu Tyr Gln 315

946 GGT GGG ATG GTT AAC TCG CAG GTA AAA CAG GCG CAG TAT AAC TTC 990
316 Gly Gly Met Val Asn Ser Gln Val Lys Gln Ala Gln Tyr Asn Phe 330

991 GTC GGC GCA AGC GAA CAG CTG GAA AGC GCG CAC CGT AGC GTG GTG 1035
331 Val Gly Ala Ser Glu Gln Leu Glu Ser Ala His Arg Ser Val Val 345

1036 CAG ACC GTA CGT TCT TCC TTT AAC AAT ATT AAC GCC TCC ATC AGC 1080
346 Gln Thr Val Arg Ser Ser Phe Asn Asn Ile Asn Ala Ser Ile Ser 360

1081 AGC ATC AAC GCG TAT AAA CAG GCG GTC GTT TCC GCG CAA AGT TCT 1125
361 Ser Ile Asn Ala Tyr Lys Gln Ala Val Val Ser Ala Gln Ser Ser 375

1126 TTG GAT GCC ATG GAA GCC GGT TAC TCG GTC GGT ACA CGT ACC ATT 1170
376 Leu Asp Ala Met Glu Ala Gly Tyr Ser Val Gly Thr Arg Thr Ile 390

1171 GTT GAC GTA CTG GAT GCC ACC ACC ACT CTG TAT GAT GCC AAG CAG 1215
391 Val Asp Val Leu Asp Ala Thr Thr Thr Leu Tyr Asp Ala Lys Gln 405

1216 CAA CTG GCC AAC GCG CGT TAT ACC TAT TTG ATT AAT CAG TTA AAT 1260
 
406 Gln Leu Ala Asn Ala Arg Tyr Thr Tyr Leu Ile Asn Gln Leu Asn 420

1261 ATC AAA TAT GCG CTC GGT ACG CTG AAC GAG CAG GAT CTG CTC GCG 1305
421 Ile Lys Tyr Ala Leu Gly Thr Leu Asn Glu Gln Asp Leu Leu Ala 435

1306 CTT AAC AGT ACG TTG GGT AAA CCT ATC CCG ACG TCG CCG GAA AGC 1350
436 Leu Asn Ser Thr Leu Gly Lys Pro Ile Pro Thr Ser Pro Glu Ser 450

1351 GTA GCG CCG GAA ACG CCA GAT CAG GAT GCT GCC GCA GAC GGT TAT 1395
451 Val Ala Pro Glu Thr Pro Asp Gln Asp Ala Ala Ala Asp Gly Tyr 465

1396 AAT GCT CAT AGC GCC GCG CCA GCA GTA CAG CCG ACC GCC GCT CGC 1440
466 Asn Ala His Ser Ala Ala Pro Ala Val Gln Pro Thr Ala Ala Arg 480

1441 GCC AAC AGC AAT AAC GGC AAT CCA TTC CGG CAT TGA 1476
481 Ala Asn Ser Asn Asn Gly Asn Pro Phe Arg His End 491
 or a DNA or a RNA sequence and coding the same sequence of amino acids as said coding sequence or a DNA or a RNA sequence complimentary to the said coding sequence with no mismatches between opposed nucleotides.

3. The molecule of Claim 2, wherein said molecule is DNA.

4. The molecule of claim 3, wherein said molecule contains the amino acid sequence.

5. The molecule of claim 2, wherein said molecule is RNA and contains a sequence corresponding or complimentary to said ST50 gene sequence.

6. The molecule of claim 1, wherein said sequence is preceded by a functional promoter sequence 5' to the said sequence.

7. The molecule of claim 6, wherein at least one copy of said sequence is present in functioning recombinant DNA or RNA vector.

8. A genetically engineered microorganism, wherein said microorganism comprises the vector of claim 7.

9. The microorganism of claim 8, wherein said microorganism is of bacterial, fungal or viral in origin.

10. A genetically engineered cell line, wherein said cell line comprises the vector of claim 7.

11. The cell line of claim 10, wherein said cell line is of animal and insect origin.

12. An isolated oligonucleotide of more than 5 consecutive nucleotides selected from nucleotide sequences consisting of a first DNA sequence: - 
1 ATG CAA ATG AAG AAA TTG CTC CCC ATC CTT ATC GGC CTG AGC CTG 45
1 Met Gln Met Lys Lys Leu Leu Pro Ile Leu Ile Gly Leu Ser Leu 15

46 TCG GGG TTC AGC ACA CTA AGC CAG GCA GAG AAC CTG ATG CAA GTT 90
16 Ser Gly Phe Ser Thr Leu Ser Gln Ala Glu Asn Leu Met Gln Val 30

91 TAT CAG CAA GCA CGC CTG AGC AAC CCG GAA TTG CGT AAA TCC GCT 135
31 Tyr Gln Gln Ala Arg Leu Ser Asn Pro Glu Leu Arg Lys Ser Ala 45

136 GCC GAT CGC GAT GCT GCA TTC GAA AAA ATT AAC GAA GCG CGT AGT 180
46 Ala Asp Arg Asp Ala Ala Phe Glu Lys Ile Asn Glu Ala Arg Ser 60

181 CCT TTA CTG CCG CAA CTG GGT TTA GGT GCC GAC TAC ACC TAC AGC 225
61 Pro Leu Leu Pro Gln Leu Gly Leu Gly Ala Asp Tyr Thr Tyr Ser 75

226 AAC GGT TAT CGC GAT GCG AAC GGT ATC AAC TCC AAT GAA ACC AGC 270
76 Asn Gly Tyr Arg Asp Ala Asn Gly Ile Asn Ser Asn Glu Thr Ser 90

271 GCT TCT CTG CAA TTA ACG CAG ACG CTA TTT GAT ATG TCG AAA TGG 315
91 Ala Ser Leu Gln Leu Thr Gln Thr Leu Phe Asp Met Ser Lys Trp 105

316 CGT GGG CTC ACC CTG CAA GAA AAA GCA GCA GGC ATT CAG GAT GTC 360
106 Arg Gly Leu Thr Leu Gln Glu Lys Ala Ala Gly Ile Gln Asp Val 120
 
361 ACC TAT CAG ACC GAT CAG CAG ACG CTG ATC CTC AAT ACC GCG AAC 405
121 Thr Tyr Gln Thr Asp Gln Gln Thr Leu Ile Leu Asn Thr Ala Asn 135

406 GCG TAT TTT AAG GTA TTG AAC GCT ATT GAT GTG CTT TCC TAT ACC 450
136 Ala Tyr Phe Lys Val Leu Asn Ala Ile Asp Val Leu Ser Tyr Thr 150

451 CAG GCG CAA AAA GAG GCT ATC TAC CGT CAG TTA GAT CAA ACG ACG 495
151 Gln Ala Gln Lys Glu Ala Ile Tyr Arg Gln Leu Asp Gln Thr Thr 165

496 CAA CGT TTT AAC GTG GGT CTG GTC GCC ATT ACC GAC GTG CAA AAC 540
166 Gln Arg Phe Asn Val Gly Leu Val Ala Ile Thr Asp Val Gln Asn 180

541 GCC CGT GCG CAA TAT GAT ACC GTA CTG GCG AAT GAA GTG ACC GCC 585
181 Ala Arg Ala Gln Tyr Asp Thr Val Leu Ala Asn Glu Val Thr Ala 195

586 CGC AAC AAC CTG GAT AAC GCG GTA GAA GAG CTG CGC CAG GTA ACC 630
196 Arg Asn Asn Leu Asp Asn Ala Val Glu Glu Leu Arg Gln Val Thr 210

631 GGC AAT TAT TAC CCG GAG CTG GCG TCG CTT AAC GTC GAG CAT TTT 675
211 Gly Asn Tyr Tyr Pro Glu Leu Ala Ser Leu Asn Val Glu His Phe 225

676 AAA ACC GAC AAA CCC AAA GCT GTT AAT GCG CTG TTG AAG GAA GCG 720
226 Lys Thr Asp Lys Pro Lys Ala Val Asn Ala Leu Leu Lys Glu Ala 240

721 GAA AAC CGT AAC CTG TCG CTG TTG CAG GCG CGT TTA AGT CAG GAT 765
241 Glu Asn Arg Asn Leu Ser Leu Leu Gln Ala Arg Leu Ser Gln Asp 255

766 CTG GCG CGC GAG CAA ATC CGT CAG GCG CAG GAT GGT CAC CTG CCG 810
256 Leu Ala Arg Glu Gln Ile Arg Gln Ala Gln Asp Gly His Leu Pro 270

811 ACG CTG AAT TTA ACG GCC TCA ACC GGC ATT TCT GAT ACC TCT TAT 855
271 Thr Leu Asn Leu Thr Ala Ser Thr Gly Ile Ser Asp Thr Ser Tyr 285

856 AGC GGT TCT AAA ACC AAC TCC ACC CAG TAC GAC GAT AGC AAC ATG 900
286 Ser Gly Ser Lys Thr Asn Ser Thr Gln Tyr Asp Asp Ser Asn Met 300

901 GGG CAG AAT AAA ATC GGC CTT AAC TTC TCC CTG CCG CTG TAT CAA 945
301 Gly Gln Asn Lys Ile Gly Leu Asn Phe Ser Leu Pro Leu Tyr Gln 315

946 GGT GGG ATG GTT AAC TCG CAG GTA AAA CAG GCG CAG TAT AAC TTC 990
316 Gly Gly Met Val Asn Ser Gln Val Lys Gln Ala Gln Tyr Asn Phe 330

991 GTC GGC GCA AGC GAA CAG CTG GAA AGC GCG CAC CGT AGC GTG GTG 1035
331 Val Gly Ala Ser Glu Gln Leu Glu Ser Ala His Arg Ser Val Val 345

1036 CAG ACC GTA CGT TCT TCC TTT AAC AAT ATT AAC GCC TCC ATC AGC 1080
346 Gln Thr Val Arg Ser Ser Phe Asn Asn Ile Asn Ala Ser Ile Ser 360

1081 AGC ATC AAC GCG TAT AAA CAG GCG GTC GTT TCC GCG CAA AGT TCT 1125
 
361 Ser Ile Asn Ala Tyr Lys Gln Ala Val Val Ser Ala Gln Ser Ser 375

1126 TTG GAT GCC ATG GAA GCC GGT TAC TCG GTC GGT ACA CGT ACC ATT 1170
376 Leu Asp Ala Met Glu Ala Gly Tyr Ser Val Gly Thr Arg Thr Ile 390

1171 GTT GAC GTA CTG GAT GCC ACC ACC ACT CTG TAT GAT GCC AAG CAG 1215
391 Val Asp Val Leu Asp Ala Thr Thr Thr Leu Tyr Asp Ala Lys Gln 405

1216 CAA CTG GCC AAC GCG CGT TAT ACC TAT TTG ATT AAT CAG TTA AAT 1260
406 Gln Leu Ala Asn Ala Arg Tyr Thr Tyr Leu Ile Asn Gln Leu Asn 420

1261 ATC AAA TAT GCG CTC GGT ACG CTG AAC GAG CAG GAT CTG CTC GCG 1305
421 Ile Lys Tyr Ala Leu Gly Thr Leu Asn Glu Gln Asp Leu Leu Ala 435

1306 CTT AAC AGT ACG TTG GGT AAA CCT ATC CCG ACG TCG CCG GAA AGC 1350
436 Leu Asn Ser Thr Leu Gly Lys Pro Ile Pro Thr Ser Pro Glu Ser 450

1351 GTA GCG CCG GAA ACG CCA GAT CAG GAT GCT GCC GCA GAC GGT TAT 1395
451 Val Ala Pro Glu Thr Pro Asp Gln Asp Ala Ala Ala Asp Gly Tyr 465

1396 AAT GCT CAT AGC GCC GCG CCA GCA GTA CAG CCG ACC GCC GCT CGC 1440
466 Asn Ala His Ser Ala Ala Pro Ala Val Gln Pro Thr Ala Ala Arg 480

1441 GCC AAC AGC AAT AAC GGC AAT CCA TTC CGG CAT TGA 1476
481 Ala Asn Ser Asn Asn Gly Asn Pro Phe Arg His End 491
 and DNA and RNA sequences encoding same sequences of the recombinant ST50 protein as well as the DNA and RNA sequences complimentary to the said first sequence with no mismatches between opposing nucleotides.

13. A polynucleotide sequence comprising of one or more genes which encodes for one or more recombinant ST50 proteins or structural and/or functional equivalents thereof which induces anti-typhoid antibodies or protective immune responses upon introduction into vertebrate tissues.

14. A vaccine which comprises of recombinant ST50 proteins or parts thereof which induces immune responses against typhoid.

15. A DNA vaccine which comprises of polynucleotide sequence of Claim 13 with our without a pharmaceutically acceptable carrier which induces protection in a vertebrate against typhoid.
